(19) Europäisches Patentamt
European Patent Office
Office européen des brevets

(11) Publication number: **0 070 131 A1**

(12) **EUROPEAN PATENT APPLICATION**

(21) Application number: 82303487.1

(22) Date of filing: 02.07.82

(51) Int. Cl.³: C 07 F 9/38
C 07 F 9/65, A 61 K 31/675

(30) Priority: 09.07.81 AU 9682/81
13.11.81 AU 1560/81
08.04.82 AU 3542/82

(43) Date of publication of application:
19.01.83 Bulletin 83/3

(84) Designated Contracting States:
CH DE FR GB IT LI SE

(71) Applicant: ANALYTICAL RESEARCH PHARMACEUTICALS PTY. LTD.
792 Elizabeth Street
Melbourne Victoria(AU)

(72) Inventor: Woods, Ross Alexander
3/10 Cook Street
West Brunswick Victoria(AU)

(72) Inventor: Caselli, Anthony Silvio
3 Nelson Court
Avondale Heights Victoria(AU)

(74) Representative: Turner, Paul Malcolm
PAUL M. TURNER AND COMPANY European Patent Attorneys 47 Marylebone Lane
London W1M 6DL(GB)

(54) **Phosphonoalkanoylamino acids.**

(57) Certain new phosphonoalkanoylamino acids of the formula (I) herein and their pharmaceutically acceptable salts exhibit improved antihypertensive properties and are particularly useful for the reduction of angiotensin related hypertension.

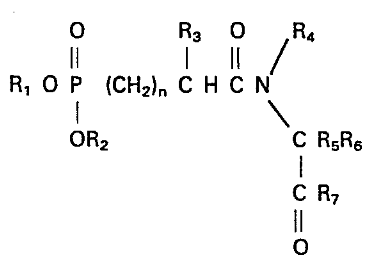

in which $R_1$ and $R_2$, which are the same or different, are each selected from the group consisting of a hydrogen atom, an optionally substituted lower alkyl, a lower alkenyl, an optionally substituted aryl and an optionally substituted aralkyl radical;

n is 0 or 1;

$R_3$ is a hydrogen atom, an optionally substituted lower alkyl, a lower alkenyl, an optionally substituted aryl or an optionally substituted aralkyl group; and

(a) $R_4$ is a hydrogen atom, a lower alkyl or a phenyl-lower alkyl radical;

$R_5$ and $R_6$, which are the same or different, are each selected from the group consisting of a hydrogen atom, an optionally substituted lower alkyl or an aryl radical; and

$R_7$ is selected from the group consisting of $-OR_8$, $-NR_9$ or $-SR_{10}$, wherein $R_8$ and $R_{10}$ are each selected from the group consisting of a hydrogen atom, a lower alkyl, an optionally substituted aryl, or an optionally substituted aralkyl radical and $R_9$ is a hydrogen atom, a hydroxy or lower alkyl radical or the residue of an – amino acid; or

(b) $R_4$ and $R_5$ are part of a four-, five- or six-membered optionally substituted heterocyclic ring as a connected bridge of 2 to 4 atoms of carbon or carbon and either nitrogen, sulphur or oxygen; and

$R_6$ and $R_7$ are each as defined in (a); with the proviso that when $R_3$ is a hydrogen atom, n is 0, the heterocyclic ring is an unsubstituted pyrrolidine, $R_6$ is a hydrogen atom, and $R_7$ is $OR_8$, at least one of $R_1$, $R_2$ and $R_8$ is an optionally substituted aryl radical; or

(c) $R_5$ and $R_6$ are selected to form a side chain of α-amino acid, where $N(R_4)\ CR_5R_6COR_7$ is an α-amino acid residue; with the proviso that (i) when $R_3$ is hydrogen and n is 0, the

./...

EP 0 070 131 A1

α-amino acid residue is not alanine, aspartic acid, glutamic acid, glycine or phenylalanine, and (ii) when $R_3$ is hydrogen and n is 1, the α-amino acid residue is not aspartic acid; or (d) $R_5$ and $R_6$ are part of a cycloalkyl ring of from 3 to 6 carbon atoms; and

$R_4$ and $R_7$, where appropriate, are each as defined in (a); or a pharmaceutically acceptable salt of the compounds of the formula (I).

PHOSPHONOALKANOYLAMINO ACIDS

This invention relates to new phosphonoalkanoylamino acids of the general formula (I)

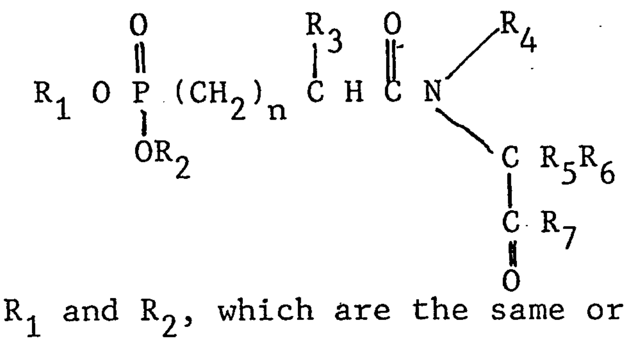

in which $R_1$ and $R_2$, which are the same or different, are each selected from the group consisting of a hydrogen atom, an optionally substituted lower alkyl, a lower alkenyl, an optionally substituted aryl and an optionally substituted aralkyl radical;
n is 0 or 1;
$R_3$ is a hydrogen atom, an optionally substituted lower alkyl, a lower alkenyl, an optionally substituted aryl or an optionally substituted aralkyl group; and

(a) $R_4$ is a hydrogen atom, a lower alkyl or a phenyl-lower alkyl radical;

$R_5$ and $R_6$, which are the same or different, are each selected from the group consisting of a hydrogen atom, an optionally substituted lower alkyl or an aryl radical; and

$R_7$ is selected from the group consisting of $-OR_8$, $-NR_9$ or $-SR_{10}$, wherein $R_8$ and $R_{10}$ are each selected from the group consisting of a hydrogen atom, a lower alkyl, an optionally substituted aryl, or an optionally substituted aralkyl radical and $R_9$ is a hydrogen atom, a hydroxy or lower alkyl radical or the residue of an $\alpha$ - amino acid; or

(b) $R_4$ and $R_5$ are part of a four-, five- or six-membered optionally substituted heterocyclic ring as a connected bridge of 2 to 4 atoms of carbon or carbon and either

nitrogen, sulphur or oxygen; and

$R_6$ and $R_7$ are each as defined in (a); with the proviso that when $R_3$ is a hydrogen atom, n is 0, the heterocyclic ring is an unsubstituted pyrrolidine, $R_6$ is a hydrogen atom, and $R_7$ is $OR_8$, at least one of $R_1$, $R_2$ and $R_8$ is an optionally substituted aryl radical; or

(c) $R_5$ and $R_6$ are selected to form a side chain of an $\alpha$- amino acid, where $N(R_4)\ CR_5R_6COR_7$ is an $\alpha$- amino acid residue, for example one classified as essential with respect to its growth effect on rates, with the proviso that (i) when $R_3$ is hydrogen and n is 0, the - amino acid residue is not alanine, aspartic acid, glutamic acid, glycine or phenylalanine, and (ii) when $R_3$ is hydrogen and n is 1, the $\alpha$- amino acid residue is not aspartic acid; or

(d) $R_5$ and $R_6$ are part of a cycloalkyl ring of from 3 to 6 carbon atoms (as, for example, when the $N(R_4)CR_5R_6COR_7$ moiety of (c) is the 1-aminocyclopentanecarboxylate residue); and

$R_4$ and $R_7$, where appropriate, are each as defined in (a); and to pharmaceutically acceptable salts of compounds of the formula (I).

As used herein, the expression "lower alkyl" includes straight chain, branched chain and cycloalkyl groups having up to seven carbon atoms: for example, methyl, ethyl propyl, isopropyl, butyl, sec. butyl, tert. butyl, cyclopentyl and cyclohexyl. In the straight and branched chain aliphatic hydrocarbons the $C_1$ to $C_4$ members, and especially the $C_1$ to $C_2$ members are preferred. Cyclopentyl or cyclohexyl radicals are the preferred cycloalkyl groups.

The salts include the metal salts, preferably the alkali and alkaline earth metal salts, and especially the sodium, potassium, lithium and calcium salts. Other salts that may be mentioned are the ammonium and quarternary ammonium salts.

When $R_4$ ad $R_5$ form part of a connected bridge, and $R_6$ is a hydrogen atom, the heterocyclic ring can contain unsaturation as exemplified by moieties such as

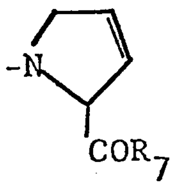

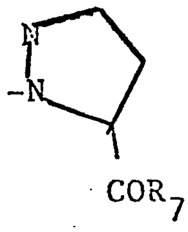

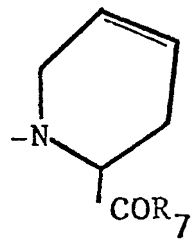

and/or can be substituted at any of the carbon atoms in the bridge as exemplified by moieties such as

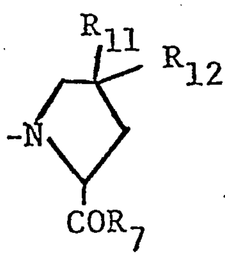

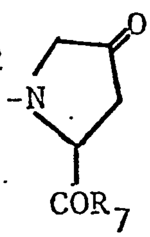

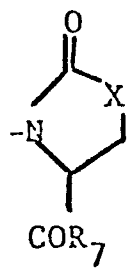

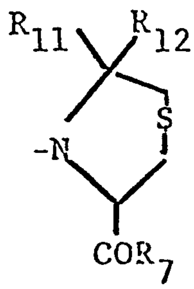

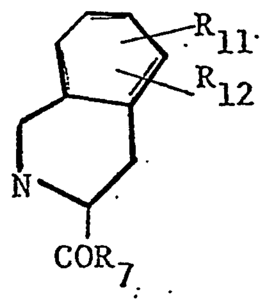

wherein X is $CH_2$, O, S or NH and $R_{11}$ and $R_{12}$, which are the same or different, are each selected from the group consisting of a hydrogen atom, a halogen atom, a hydroxy, lower alkyl, a cycloalkyl, an optionally substituted aryl, an optionally substituted aralkyl an alkyoxy, an aryloxy or a sulphenyl radical. When a sulphur atom is present in the heterocyclic ring or substituents it can be in the oxidation state of 2, 4 or 6 as a sulphide, sulphoxide or sulphone.

When $R_4$ and $R_5$ are bridged, a five-membered heterocyclic ring is preferred, for example thiazolidine or pyrrolidine and especially thiazolidine.

When n is 1 and $N(R_4)CR_5R_6COR_7$ is the residue of an $\alpha$ - amino acid, arginine, phenylalanine, leucine, methionine and lysine are preferred.

It is to be understood that this invention encompasses the isomeric forms of the compounds as well as racemic

mixtures thereof. The pure isomeric forms can be synthesized from the corresponding optically active constituents or the racemic mixtures separated by techniques well known in the art.

The compounds of this invention are antihypertensive agents and are particularly useful for the reduction of angiotensin related hypertension. Present indications are that they are also likely to be useful as antibacterial, anticancer and antiviral agents.

Angiotensin converting enzyme is a dipeptidyl carboxypeptidase which catalyzes the hydrolysis of the C-terminal l-histidyl-l-leucine residue of angiotensin I to produce the octapeptide angiotensin II, a powerful vasoconstrictor. Inhibitors of angiotensin converting enzyme can reduce or alleviate hypertension. Based on the similarity of this enzyme to the zinc dependent carboxypeptidase A, a series of angiotensin converting enzyme inhibitors containing a mercaptoalkanoyl moiety were developed (D.W. Cushman, H.S. Cheung, E.F. Sabo and M.A. Ondetti, Biochem., 16, 5484 (1977)). In compounds of this type the sulphydryl group chelates with the zinc atom at the active site of the metalloenzyme. Phosphoryl groups are also known to provide a strong zinc binding function in the inhibition of metalloenzymes (C. Kam, N. Nishino and J.C. Powers, Biochem., 18, 3032 (1979)). Several N-phosphoryl dipeptides (R.E.Galardy, Biochem. Biophys. Res. Chom., 97, 94 (1980); E.D. Thorsett, A.A. Patchett, E.E. Harris and A.L. Maycock, European Patent No.
), phosphonoacyl prolines (M.A. Ondetti and E.W. Petrillo, U.S. Patent No. 4,151,172 Apr. 24 1979) and phosphinylacyl prolines (E.W. Petrollo, U.S. Patent No.
) are known to inhibit angiotensin converting enzyme.

The preferred compounds of formula (I), which are angiotensin converting enzyme inhibitors, are those wherein

n is 1; at least one of R, and $R_2$ is a hydrogen atom; $R_3$ is a methyl group; $R_4$ and $R_5$ form part of a pyrrolidine or thiazolidine ring and especially thiazolidine; $R_6$ is a hydrogen atom; and $R_7$ forms part of a carboxylic acid or ester ($OR_8$). The phosphonoalkanoyl amino acids of this invention are superior to the mercaptoalkanoyl amino acid inhibitors, because they lack the toxicity associated with the presence of the sulphydryl group (Lancet, 1980, 129) Compounds of the formula (I), which contain a phosphorus carbon bond, are more stable than the N-phosphorylated dipeptides and related compounds of the formula

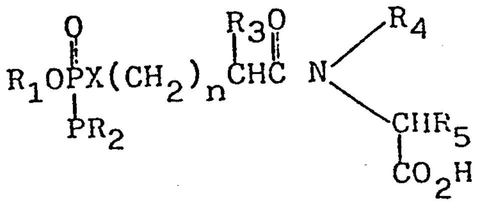

where X is NH, O or S, which are susceptible to hydrolysis (see T.C. Bruice and S.J. Benkovic "Bioorganic Mechanisms" Vol II, W.A. Benjamin, New York, 1966, Chapter 5). Of the known phosphonoacyl prolines, 1-(phosphonoacetyl)-1-proline was the most potent inhibitor of angiotensin converting enzyme. Either lengthening or substituting the acyl side chain diminished the activity (E.W. Petrillo, M.A. Ondetti, D.W. Cushman, E.R.Weaver and J.E. Heikes, Abst. 176th Meeting Amer. Chem. Soc., Sept 1978, MEDI-27). Thus U.S. Patent No. 4,151,172, Apr. 24, 1979 claims that n equals 0 is preferred for compounds of the general formula I, where $R_4$ and $R_5$ forms a pyrrolidine ring. However, phosphonoacyl prolines and the new compounds described herein are even more effective antihypertensives when the acyl side chain is both lengthened as well as substituted i.e. compounds of formula I where n is 1 and $R_3$ is methyl are preferred. Incorporation of a thiazolidine ring into these agents also represents an advantage over the proline series by increasing the lipid solubility of the compound and increasing penetration across the blood brain barrier.

When n is 1, the compounds of this invention can be prepared by (I) reacting a trialkyl or triallylphosphite of the formula

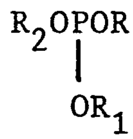

with a 2-substituted acrylic acid of the formula

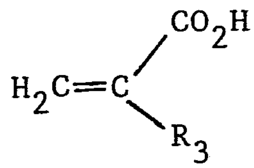

to form a triester of the formula

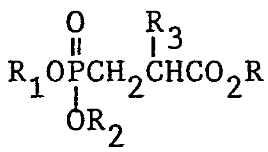 (II) ;

wherein $R_1$, $R_2$ and $R_3$ are each as defined in claim 1 and R is a lower alkyl, aryl, or aralkyl radical; (II) hydrolysing the thus formed triester with a base to form a diester of the formula

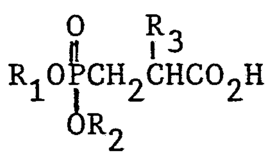 (III) ;

and (III) reacting the thus formed diester with a compound of the formula

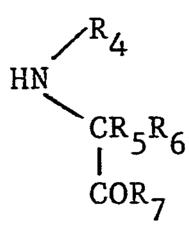 (IV) ;

in the presence of a condensing agent, thereby to form a compound according to claim 1.

The 2 - substituted acrylic acid may be,for example, methacrylic acid, and the condensing agent dicyclohexyl-carbodiimide.

The trialkylphosphites can be prepared by standard techniques; see "Vogels Textbook of Practical Organic Chemistry", Fourth Edition, Longmans, 1978, page 405, and T.R.Herrin, J.S. Fairgrieve, R.R.Bower, N.L. Shiplowitz, and J.C.H. Mao, J.Med. Chem., 20, 660 (1977).

Compounds of the formula IV, where $R_7$ does not contain a hydroxyl, hydroxyamino or sulphydryl radical, are preferred. Especially preferred are compounds where $R_7$ forms a lower alkyl or aralkyl ester such as methyl, ethyl, t-butyl or phenylmethyl, which can be easily removed by base or acid catalyzed hydrolysis, or by catalytic hydrogenation as exemplified in "Protective Groups in Organic Chemistry Ed. J.F.W. McOmie, Plenum Press London, 1973 and the references therein.

Compounds of the formula (III) can also be activated, for example by formation of the acid chloride. Subsequent acylation of compounds of the formula (IV) with this activated acid then yields compounds of the formula (I).

When n is 1, compounds of the formula I can also be prepared by carrying out the condensation of the trialkylphosphite and 2 - substituted acrylic acid in the presence of an amine of the general formula (IV) where $R_7$ is preferably an alkoxy or amino group. This procedure avoids the intermediate hydrolysis and acylation reactions of the two step process.

Compounds of the formula (IV) where $R_7$ is $OR_8$, can be prepared from the corresponding amino acids by the standard techniques of esterification, as exemplified by G.Jolles, G. Poiget, J.Robert, B.Terlain and J.P. Thomas, Bull. Soc. Chem. France, 1965, 2252; R.E.Neuman and E.L. Smith, J.Biol. Chem., 193, 97 (1951); D.J. Hanahan and R. Vercamer, J.Amer. Chem. Soc., 76, 1906 (1954) and S Ratner and H.T.Clarke, J. Amer. Chem. Soc., 59, 200 (1937).

When $R_7$ in formula (1V) is $NHR_9$ or $SR_{10}$ the compounds can be prepared by reaction of the corresponding

amine or sulphydryl compound with lower alkyl esters, acylhalides or anhydrides of the amino acid, as exemplified by R.W. Chambers and F.H. Carpenter, J.Amer. Chem. Soc., 77, 1522 (1955) and D.P.N. Satchell, Quart. Rev., 17, 182 (1963); or by reacting amino acids in the presence of a coupling reagent like dicyclohexylcarbodiimide. During this process other reactive centres in the amino acid may be protected, with easily removable groups, by the standard techniques utilized in peptide chemistry. Compounds of the Formula IV, where $R_4$ and $R_5$ form part of a heterocyclic ring which is substituted or contains an alkene group can be prepared by a variety of procedures including the condensation of cysteine with aldehydes or ketones as exemplified by B.Paul and W. Korytnyk, J. Med. Chem., 19, 1002 (1976) and V.M. Kulkarni and H.P. Tipnis, Curr. Sci., 41, 637 (1972); reduction of pyrrole carboxylic acids or alkylation, halogenation, oxidation or dehydration of hydroxy prolines, for example see S.S. Kerwar, A.M. Felix, R.J. Marcel, I. Tsai and R.A. Salvador, J. Biol. Chem., 251, 503 (1976) and R.H. Andreatta, V.Nair, A.V. Robertson and W.R.J. Simpson, Aust. J. Chem., 20, 1494 (1967) and other standard processes well known in the art. When a sulphide group is present in compounds of the formula (I) or (IV), for example as a thiazolidine ring, it can be converted to the corresponding sulphoxide or sulphone by oxidation using standard techniques as exemplified by "Organic Functional Group Preparations" S.R. Sandler and W.Karo, Academic Press, London, 1968, Vol. 1, Chapters 19 and 20.

When n is zero, the compounds of this invention can be prepared by substituting intermediates of the formula (V) (wherein R is lower alkyl, aryl or aralkyl) for compounds of the formula (II):

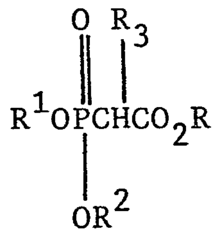

(V);

Compounds of the formula (V) can be prepared by the standard Michaelis-Arbuzov reactions of a trialkyl- or triallylphosphite and an -haloester as illustrated by L. Maier and M.M. Crutchfield, Phosphorous and Sulfur, 5, 45 (1978) and the references therein.

The alkoxyphosphinyl groups in compounds of the general formula (I) can be selectively converted to the corresponding phosphoric acids by hydrolysis in acid, catalytic hydrogenation or by treatment with trimethyl-halosilanes, e.g. as illustrated by T.R. Herrin, J.S. Fairgrieve, R.R.Bower, N.L. Shipkowitz and J.C.H. Mao, J. Med. Chem., 20, 660 (1977); C.E. McKenna and J. Schmidhauser, J.C.S. Chem. Comm., 1979, 739 and the references therein.

The compounds of the general formula I, wherein $R_7$ is an ester group, can also be prepared by esterification of the corresponding carboxylic acids, using standard techniques. Esterification of this carboxylic acid can also be carried out with simultaneous acid hydrolysis of the alkoxyphosphinyl groups. Similarly when $R_7$ in formula I is part of a carboxylic acid or ester group, it can be converted to an amide, hydroxamic acid or thioester by standard procedures.

For antihypertensive activity a single dose, or preferably two to four divided daily doses, provided on a basis of 0.5 to 500 mg. per kilogram per day, is appropriate to bring about a reduction in elevated blood pressure. The animal model experiments described by Patchett et al., Nature, 288, 280-283, (1980) provide a valuable guide.

The composition is preferably administered subcutaneously, intramusculary, intravenously or intraperitoneally, but it can also be administered orally with a dose of 1 to 500 mg per kilogram per day, preferably about 10 to 100 mg per kilogram per day. The compound or compounds of formula

I can be formulated as tablets, capsules or elixirs for oral administration. Sterile solutions or suspensions can be used for parenteral use.

About 1.0 to 500 mg of a compound or compounds of formula I can be compounded with a physiologically acceptable vehicle, carrier, excipient, binder, preservative, stabilizer, flavour, etc., in a conventional unit dosage form as called for by accepted pharmaceutical practice. The amount of active substance is selected so as to provide a dosage in the range indicated.

The following examples are illustrative of the invention, or of intermediates which may lead to the invention, or of methods used and represent preferred embodiments. All temperatures are in degress Celsius.

## EXAMPLE 1

### DIETHYL 2-CARBOETHOXYPROPYLPHOSPHONATE

A mixture of triethylphosphite (6 g, 0.0361 mole), methacrylic acid (3 g, 0.0349 mole) and hydroquinone (0.09 g) was heated at reflux for six hours. The crude product was distilled to yield diethyl 2-carboethoxypropylphosphonate as a colourless liquid (bp 120$^o$, 1mm) (yield 6.1 g, 78%); $M^+$ 252; ir (neat) 2970, 1725, 1450, 1380, 1240, 1200, 1160, 1020, 960 and 830 $cm^{-1}$; pmr ($CDCl_3$) $\delta$ 4.1 (m, 6 H), 2.8 (bm, 1 H), 2.4 - 1.6 (bm, 2 H) and 1.3 (m, 12 H) ppm.

## EXAMPLE 2

### DIMETHYL 2-CARBOMETHOXYPROPYLPHOSPHONATE

A mixture of trimethylphosphite (50 $cm^3$, 0.424 mole), methacrylic acid (36 $cm^3$, 0.424 mole) and hydroquinone (0.2 g) was heated at reflux for six hours. The crude product was distilled to yield dimethyl 2-carbomethoxypropylphosphonate as a colourless liquid (bp 112°, 1mm) (yield 58 g, 65%); ir (neat) 2950, 1736, 1455, 1240, 1170, 1040 and 835 $cm^{-1}$; pmr ($CDCl_3$) $\delta$ 3.7 (d, 6 H), 3.6 (s, 3 H), 2.8 (bm, 1 H) 2.1 (bm, 2 H) and 1.3 (d, 3 H) ppm.

## EXAMPLE 3

## DIMETHYL 2-CARBOXYPROPYLPHOSPHONATE

A cold solution of sodium hydroxide (2.1 g, 0.0525 mole) in water (40 $cm^3$) was added to a solution of dimethyl 2-carbomethoxpropylphosphonate (11.4 g, 0.0542 mole) in cold water (10 $cm^3$). The reaction was maintained in an ice bath for fifteen minutes and finally stirred at room temperature for three hours. The solution was extracted with water saturated ethyl acetate (2 x 50 $cm^3$). The aqueous layer was separated and the solvent removed in vacuo below 40°. The residue was acidified with ice cold hydrochloric acid (2:1, $HCl:H_2O$), saturated with sodium chloride and extracted with wet ethyl acetate (4 x 75 $cm^3$). The combined ethyl acetate fractions were concentrated in vacuo, filtered and evaporated to yield dimethyl 2-carboxypropylphosphonate, (9.69 g, 0.0494 mole, 94%) which was not further purified; ir (neat) 3500-2500, 1715, 1455, 1200, 1030 and 820 $cm^{-1}$; pmr ($CDCl_3$) $\delta$ 11.3, (s, 1 H), 3.7 (d, 6 H), 2.8 (bm, 1 H), 2.1 (bm, 2 H) and 1.3 (d, 3 H) ppm.

## EXAMPLE 4

## DIMETHYL 2-CARBOXYETHYLPHOSPHONATE

By replacing methacrylic acid in example 2 with acrylic acid, dimethyl 2-carbomethoxyethylphosphonate can be prepared. Hydrolysis as in example 3, yields dimethyl 2-carboxyethly-phosphonate.

EXAMPLE 5

ETHYL L-THIAZOLIDINE-4-CARBOXYLATE HYDROCHLORIDE

1-Thiazolidine-4-carboxylic acid (10 g, 0.0751 mole) was dissolved in absolute ethanol (200 $cm^3$). The solution cooled in an ice bath and dry, gaseous hydrogen chloride bubbled through for 30 minutes. The solution was heated at reflux for 1 hour and the solvent removed *in vacuo*. The residue was recrystallized from ethanol, washed with ether and dried *in vacuo* to yield ethyl 1-thiazolidine-4-carboxylate hydrochloride as a white solid (13,0 g, 0.0658 mole, 88%); pmr ($D_2O$) $\delta$ 5.1-4.6 (m, 1 H), 4.5 (s, 2 H), 4.4 (a, 2 H), 3.5 (d, 2 H) and 1.3 (t, 3 H) ppm.

EXAMPLE 6

ETHYL 3-(3-DIMETHOXYPHOSPHINYL-2-METHYL-1-OXOPROPYL)-L-THIAZOLIDINE-4-CARBOXYLATE

Method A :

Anhydrous triethylamine (2.9 $cm^3$, 0.02079 mole) was added to a solution of ethyl 1-thiazolidine-4-carboxylate hydrochloride (4.1 g, 0.02074 mole) in dry dichloromethane (50 $cm^3$). Anhydrous ether was added, the solution filtered and evaporated. The residue was dissolved in dichloromethane (20 $cm^3$) and treated with solutions of dicyclohexylcarbodiimide (4.5 g, 0.02181 mole) in dichloromethane (20 $cm^3$) and dimethyl 2-carboxypropylphosphonate (4.0 g, 0.02039 mole) in dichloromethane (10 $cm^3$) at $0^o$. The solution was stirred at room temperature overnight, filtered, washed with 1% hydrochloric acid 50 $cm^3$), water (50 $cm^3$) and dried over sodium sulphate to yield the product (6.5 g, 0.0192 mole, 92%); ir (neat) 2930, 2850, 1740, 1650, 1450, 1410, 1260, 1230, 1185, 1025, 810 and 730 $cm^{-1}$; pmr ($CDCl_3$) $\delta$ 5.0-3.9 (m, 5 H), 3.7 (d, 6 H), 3.5-2.8 (m, 3 H), 2.5-1.5 (m, 2 H) and 1.5-1.0 (m, 6 H) ppm.

Method B:

A mixture of dimethyl 2-carboxypropylphosphonate (1.06 g, 0.01 mole) and thionayl cholride (5 ml) was heated at reflux for 5 minutes. Dry hexane was added and the solent evaporated in vacuo giving dimethyl 2-chlorocarbonylpropyl-propylphosphate (2.064 g, 96%) as a colourless liquid which was not further purified; ir (neat) 2945, 1780, 1450, 1400, 1250, 1175, 1030, 945, 875, 840, 810, 735 and 675 $cm^{-1}$; pmr ($CDCl_3$) α 3.73 (d, 6 H), 3.50 - 3.00 (M. 1 H), 2.50 - 1.60 (M, 2 H), 1.46 (d, 3 H) ppm.

2-chlorocarbonylphosphonate (4.30 g, 0.02 mole) was added dropwise to a cooled mixture of 1-thiazolidine-4-carboxylic acid (2.663 g, 0.02 mole) and sodium barcarbonate (3.36 g, 0.04 mole) in water (30 ml). The mixture was stirred at 5°C for 15 minutes and at room temperature for 3 hours. The solution was extracted with ethyl acetate (3 x 50 ml), acidified with concentrated hydrochloric acid and re-extracted with ethylacetate (4 x 50 ml). The acidic extracts were dried and evaporated to give 3-(3-dimethoxyphosphinyl-2-methyl-1-oxopropyl)-1-thiazolidine-4-carboxylic acid as a clear viscous oil (3.0 g, 48%); IR (neat) 2800-2200, 1715, 1645, 1415, 1320, 1300, 1180, 1030, 865, 818 and 730 $cm^{-1}$; pmr ($CDCl_3$) δ 11.6 (bS, 1 H), 5.2 -4.0 (m, 4 H), 3/75 (d, 6 HO, 3.7 - 2.6 (m, 4 H) and 1.3 (dd, 3 H) ppm.

The product was treated with anhydrous ethanolic hydrochloric acid to yield ethyl 3-(3-dimethoxyphosphinyl-2-methyl-1-oxopropyl-1-thiazolidine-4-carboxylate.

Method C:

A solution of dimethyl 2-chlorocarboxylpropyl-phosphate (12,92 g, 0.0602 mole) in dry ether (50$cm^3$) was added dropwise over 45 minutes, to a solution of ethyl 1-thiazolidine-4-carboxylate (9.71 g, 0.0602 mole) and triethylamine (8.4 $cm^3$, 0.0602 mole) in dry ether (100$cm^3$) at 0°. The

The solution was stirred at room temperature for 24 hours, filtered and the solvent removed in vacuo. The residue was dissolved in dichloromethane (100 $cm^3$) and washed with dilute hydrochloric acid. The organic phase was dried over sodium sulphate and evaporated to yield ethyl 3-(3-dimethoxyphosphinyl-2-methyl-1-oxopropyl)-1-thiazolidine-4-carboxylate.

## EXAMPLE 7

### METHYL 3-(3-DIMETHOXYPHOSPHINYL-2-METHYL-1-OXOPROPYL) - L-THIAZOLIDINE-4-CARBOXYLATE

By substituting methyl 1-thiazolidine-4-carboxylate hydrochloride for ethyl 1-thiazolidine-4-carboxylate hydrochloride in example 6, methyl 3-(3-dimethoxyphosphinyl-2-methyl-1-oxopropyl-1-thiazolidine-4-carboxylate can be prepared; ir (neat) 2930, 1850, 1750, 1650, 1530, 1450, 1410, 1230, 1180, 1060, 1030, 810 and 750 $cm^{-1}$; pmr ($CDCl_3$) $\partial$ 5.2-4.3 (m, 3 H), 3.9-3.6 (m, 9 H), 3.5-3.0 (m, 3 H), 2.8-1.6 (m, 2 H) and 1.5-1.0 (m, 3 H) ppm.

## EXAMPLE 8

### ETHYL 3-(3-DIHYDROXYPHOSPHINYL-2-METHYL-1-OXOPROPYL)- L-THIAZOLIDINE-4-CARBOXYLATE

Dry hydrogen chloride was bubbled through a solution of ethyl 3-(3-dimethoxyphosphinyl-2-methyl-1-oxopropyl)-1-thiazolidine-4-carboxylate (7.0 g, 0.0206 mole) in absolute alcohol (150 $cm^3$) and water (0.82 $cm^3$, 0.0455 mole) for 10 minutes at 0°. The solution was stirred at room temperature for 24 hours and the solvent evaporated in vacuo to yield ethyl 3-(3-dihydroxyphosphinyl-2-methyl-1-oxopropyl)-1-thiazolidine-4-carboxylate; ir (neat) 3600-3000, 2930, 2850, 1740, 1650, 1450, 1420, 1300, 1190 and 1030 $cm^{-1}$; pmr ($CDCl_3$) $\partial$ 9.9-9.0 (bs, 2 H), 5.3-3.0 (m, 8 H), 2.6-1.5 (m, 2 H) and 1.5-1.0 (m, 6 H) ppm.

EXAMPLE 9

METHYL 3(3-DIHYDROXYPHOSPHINYL-2-METHYL-1-OXOPROPYL)-L-THIAZOLIDINE-4-CARBOXYLATE

By replacing ethyl 3-(3-dimethoxphosphinyl-2-methyl-1-oxopropyl)-1-thiazolidine-4-carboxylate in ethanol in example 8 with methyl 3-(3-dimethoxyphosphinyl-2-methyl-1-oxopropyl)-1-thiazolidine-4-carboxylate in dichloromethane, methyl 3-(3-dihydroxyphosphinyl-2-methyl-1-oxopropyl)-1-thiazolidine-4-carboxylate can be produced.

EXAMPLE 10

ETHYL 3-(3-DIHYDROXYPHOSPHINYL-2-METHYL-1-OXOPROPYL)-L-THIAZOLIDINE-4-CARBOXYLATE MONOLITHIUM SALT

A solution of ethyl 3-(3-dihydroxyphosphinyl-2-methyl-1-oxopropyl)-1-thiazolidine-4-carboxylate (12.0 g, 0.0385 mole) in water (2 L) was adjusted to pH 5.0 by addition of a 0.4 M solution of lithium hydroxide. The solution was filtered and concentrated in vacuo. The aqueous residue was lyophillized to yield the monolithium salt as an amorphous, hygroscopic white solid (12.24 g, 0.0385 mole, 100%); mp 50 - 70$^o$; ir (KBr) 3700-3150, 2980, 2930, 1735, 1640, 1460, 1420, 1370m 1300, 1195, 1035 and 950 $cm^{-1}$; pmr ($CDCl_3$) $\delta$ 5.35-3.1 (bm, 8 H), 2.30-1.60 (bm, 2 H), 1.55-1 (bm, 6 H) ppm.

EXAMPLE 11

3-(3-DIHYDROXYPHOSPHINYL-2-METHYL-1-OXOPROPYL)-L-THIAZOLIDINE-4-CARBOXYLIC ACID TRILITHIUM SALT

Three equivalents of lithium hydroxide monohydrate were added to an aqueous solution of ethyl 3-(-dihydroxyphosphinyl-2-methyl-1-oxopropyl)-1-thiazolidine-4-carboxylate. The solution was stirred at room temperature for 72 hours, filtered and lyophillized to yield the trilithium salt as an

amorphous white solid; mp >300°; ir (KBr) 3700-3060, 2980, 2930, 1620, 1420, 1335, 1205, 1050 $cm^{-1}$; pmr ($D_2O$) ∂ 5.2-3.05 (bm, 6 H); 2.20-1.55 (bm, 2 H), 1.1-1.5 (bm, 3 H) ppm.

EXAMPLE 12

ETHYL 3-(3-DIHYDROXYPHOSPHINYL-2-METHYL-1-OXOPROPYL)-L-THIAZOLIDINE-4-CARBOXYLATE MONOSODIUM SALT

By replacing lithium hydroxide monohydrate in example 10 with sodium hydroxide, the corresponding monosodium salt can be prepared.

EXAMPLE 13

LITHIUM 3-(3-DIMETHOXYPHOSPHINYL-2-METHYL-1-OXOPROPYL)-L-THIAZOLIDINE-4-CARBOXYLATE

A solution of lithium hydroxide (monohydrate) (0.605 g, 0.01444 mole) in water (10 $cm^3$) was added to a solution of methyl 3-(3-dimethoxyphosphinyl-2-methyl-1-oxopropyl)-l-thiazolidine-4-carboxylate (4.7 g, 0.01444 mole) in methanol (70 $cm^3$). The mixture was stirred at room temperature overnight and the solvent removed in vacuo to yield lithium 3-(3-dimethoxyphosphinyl-2-methyl-1-oxopropyl)-l-thiazolidine-4-carboxylate as a hygroscopic solid; ir (KBr) 2930, 2850, 1600, 1420, 1320, 1230, 1030, 850, 810 and 730 $cm^{-2}$; pmr ($CDCl_3$) ∂ 5.0-4.0, (m, 3 H), 3.7 (d, 6 H), 3.4-2.5 (m, 4 H), 2.5-1.6 (m, 2 H) and 1.5-1.0 (m, 3 H) ppm.

EXAMPLE 14

1-(3-DIHYDROXYPHOSPHINYL-2-METHYL-1-OXOPROPYL)-L-LEUCINE TRILITHIUM SALT

By substituting l-leucine ethyl ester for ethyl l-thiazolidine-4-carboxylate in example 6, and subsequent hydroysis as in examples 8 and 11 respectively, 1-(3-dihydroxyphos-

phinyl-2-methyl-1-oxopropyl)-l-leucine trilithium salt can be prepared.

EXAMPLE 15

1-(3-DIHYDROXYPHOSPHINYL-2-METHYL-1-OXOPROPYL)-L-PHENYLALANINE TRILITHIUM SALT

By substituting l-phenylalanine ethyl ester for ethyl l-thiazolidine-4-carboxylate in example 6, and subsequent hydrolysis as in examples 8 and 11 respectively, 1-(3-dihydroxyphosphinyl-2-methyl-1-oxopropyl)-l-phenylalanine trilithium salt can be prepared.

EXAMPLE 16

1-(3-DIMETHOXYPHOSPHINYL-2-METHYL-1-OXOPROPYL)-L-PROLINE AMIDE

By substituting l-proline amide for ethyl l-thiazolidine-4-carboxylate in example 6, 1-(3-dimethoxyphosphinyl-2-methyl-1-oxopropyl)-l-proline amide can be prepared.

EXAMPLE 17

ETHYL 3-(3-DIMETHOXYPHOSPHINYL-2-METHYL-1-OXOPROPYL)-2-PHENYL-L-THIAZOLIDINE-4-CARBOXYLATE

By substituting ethyl 2-phenyl-l-thiazolidine-4-carboxylate for ethyl l-thiazolidine-4-carboxylate in example 6, ethyl 3-(3-dimethoxyphosphinyl-2-methyl-1-oxopropyl)-2-phenyl-l-thiazolidine-4-carboxylate can be prepared.

EXAMPLE 18

3-(3-DIHYDROXYPHOSPHINYL-1-OXOPROPYL)-L-THIAZOLIDINE-4-CARBOXYLATE TRILITHIUM SALT

By substituting dimethyl 2-carboxyethylphosphonate

for dimethyl 2-carboxypropylphosphonate in example 6, ethyl 3-(3-dimethoxyphosphinyl-1-oxopropyl)-1-thiazolidine-4-carboxylate can be prepared. Subsequent hydrolysis as in examples 8 and 11 respectively yields 3-(3-dihydroxyphosphinyl-1-oxopropyl)-1-thiazolidine-4-carboxylate trilithium salt.

## EXAMPLE 19

## ETHYL 3-(3-DIETHOXYPHOSPHINYL-2-METHYL-1-OXOPROPYL)-L-THIAZOLIDINE-4-CARBOXYLATE

By substituting diethyl 2-carboxypropylphosphonate for dimethyl 2-carboxypropylphosphonate in example 6, ethyl 3-(3-diethoxyphosphinyl-2-methyl-1-oxopropyl)-1-thiazolidine-4-carboxylate can be prepared.

## EXAMPLE 20

## DIMETHYL 2-CARBOXYETHYLPHOSPHONATE

Ethyl 2-bromopropionate (30 g, 0.1657 mole) was added drowpwise to a solution of trimethylphosphite (40 $cm^3$ 0.3391 mole) heated at reflux. The mixture was maintained at $130^o$ for 2 hours and fractionated to yield dimethyl 2-carbethoxyethylphosphonate (7.5 g, 0.0412 mole, 24.9%) bp $80-6^o$ (0.5 mm Hg); ir (neat) 2980, 2960, 1730, 1460, 1360, 1310, 1255, 1180, 1030 and 830 $cm^{-1}$; pmr ($CDCl_3$) 4.3 (q, 2 H), 3.8 (d, 6 H), 3.4-2.2 (bm, 1 H), 1.4 (dd, 3 H, J=7 and 18 Hz) and 1.3 (t, 3 H) ppm. A solution of sodium hydroxide (1.4 g, 0.0350 mole) in water (40 $cm^3$) was added to a solution of dimethyl 2-carboethoxyethylphosphonate (7.48 g, 0.0355 mole) in water (25 $cm^3$). The solution was stirred at room temperature overnight, extracted with wet ethyl acetate and the aqueous phase concentrated in vacuo, below $40^o$. The solution was cooled, acidified with ice cold hydrochloric acid (diluted 2:1, acid: water), saturated with sodium chloride and extracted with wet ethyl acetate (4 x 50 $cm^3$). The ethyl acetate was removed in vacuo and the product recrystallized from

chloroform-ether to yield dimethyl 2-carboxyethylphosphonate as a white solid mp 118° (3.34 g, 0.0183 mole, 52%); ir (KBr) 3400-2300, 1710, 1450, 1380, 1300, 1230, 1170, 1020, 940 and 820 $cm^3$; pmr ($CDCl_3$-$CD_3OD$) 3.8 (d, 6 H), 3.3-2.2 (bm, 1 H) and 1.4 (dd, 3 H, J = 7 and 18 Hz) ppm.

## EXAMPLE 21

### ETHYL 3-(2-DIMETHOXYPHOSPHINYL-1-OXOPROPYL)-L-THIAZOLIDINE-4-CARBOXYLATE

Dimethyl 2-carboxyethylphosphonate (1.6 g, 0.00879 mole) and dicyclohexylcarbodiimide (1.82 g, 0.00882 mole) each in anhydrous acetonitrile (40 $cm^3$) were added to a solution of ethyl 1-thiazolidine-4-carboxylate (prepared from the hydrochloride by neutralization and distillation) (1.47 g, 0.00879 mole) in dry acetonitrile (10 $cm^3$) at 5°C. The reaction was stirred at room temperature for 4 days, filtered and the solvent removed *in vacuo*. The residue was dissolved in dichloromethane, filtered, washed with dilute hydrochloric acid, a saturated sodium bicarbonate solution and a saturated sodium chloride solution. The organic phase was dried over sodium sulphate and evaporated to yield ethyl 3-(2-dimethoxyphosphinyl-1-oxopropyl-1-thiazolidine-4-carboxylate as a viscous oil; (2.44 g, 0.00751 mole, 85%); ir (neat) 2960, 1740, 1655, 1450, 1405, 1255, 1185, 1030, 840 and 810 $cm^{-1}$; pmr ($CDCl_3$) ∂ 5.4-4.4 (m, 3 H), 4.2 (q, 2 H), 3.7 (d, 6 H), 3.5-2.3 (m, 3 H), 1.4 (dd, 3 H, J = 7 and 18 Hz) and 1.3 (t, 3 H) ppm.

## EXAMPLE 22

### 3-(2-HYDROXYPHOSPHINYL-1-OXOPROPYL)-L-THIAZOLIDINE-4-CARBOXYLATE TRILITHIUM SALT

Ethyl 3-2-dimethoxyphosphinyl-1-oxopropyl)-1-thiazolidine-4-carboxylate (2.32 g, 0.00714 mole) was dissolved in absolute ethanol (25 $cm^3$) and water (0.26 $cm^3$, 0.01444 mole), and hydrogen chloride bubbled through the solution,

at 5°, for 1 hour. The solution was stirred at room temperature for 7 days and the solvent removed *in vacuo*. The residue was dissolved in water and the pH adjusted to 5.0 by addition of a measured quantity of 0.4M lithium hydroxide. An additional two equivalents of lithium hydroxide was then added, the solution filtered and washed with ethyl acetate and lyophillized to yield the trilithium salt as an amorphous white solid; (2.0 g, 0.00698 mole, 97.7%) mp >300°; ir (KBr) 3600-2600, 1600, 1400, 1180, 1080 and 980 $cm^{-1}$; pmr ($D_2O$) 5.1-4.8 (m, 3 H), 3.8-2.5 (m, 3 H) and 1.3 (dd, 3 H) ppm.

## EXAMPLE 23

## METHYL 4-CHLORO-1-(3-DIMETHOXYPHOSPHINYL-2-METHYL-1-OXOPROPYL)-L-PROLINATE

A mixture of methyl 4-chloro-1-proline (2.54 g, 0.01552 mole) trimethylphosphite (1.83 $cm^3$, 0.01552 mole), methacrylic acid (1.31 $cm^3$, 0.01545 mole) and hydroquinone (200 mg) were heated at 120° for 7 hours. The product was dissolved in dichloromethane (100 $cm^3$), washed with dilute hydrochloric acid followed by saturated sodium bicarbonate, dried over sodium sulphate and evaporated to yield methyl 4-chloro-1-(3-dimethoxyphosphinyl-2-methyl-1-oxopropyl)-1-prolinate.

CLAIMS

1. A phosphonoalkyanoylamino acid of the general formula (I)

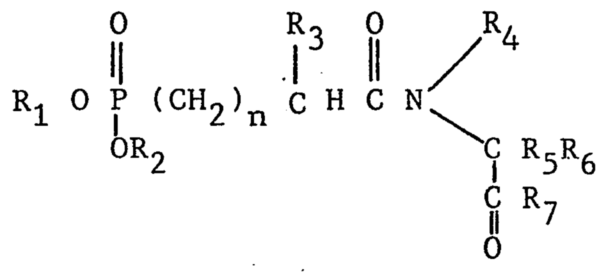

in which $R_1$ and $R_2$, which are the same or different, are each selected from the group consisting of a hydrogen atom, an optionally substituted lower alkyl, a lower alkenyl, an optionally substituted aryl and an optionally substituted aralkyl radical;

n is 0 or 1;

$R_3$ is a hydrogen atom, an optionally substituted-lower alkyl, a lower alkenyl, an optionally substituted aryl or an optionally substituted aralkyl group; and

(a) $R_4$ is a hydrogen atom, a lower alkyl or a phenyl-lower alkyl radical;

$R_5$ and $R_6$, which are the same or different, are each selected from the group consisting of a hydrogen atom, an optionally substituted lower alkyl or an aryl radical; and

$R_7$ is selected from the group consisting of $-OR_8$, $-NR_9$ or $-SR_{10}$, wherein $R_8$ and $R_{10}$ are each selected from the group consisting of a hydrogen atom, a lower alkyl, an optionally substituted aryl, or an optionally substituted aralkyl radical and $R_9$ is a hydrogen atom, a hydroxy or lower alkyl radical or the residue of an - amino acid; or

(b) $R_4$ and $R_5$ are part of a four-, five- or six-membered optionally substituted heterocyclic ring as a connected bridge of 2 to 4 atoms of carbon or carbon and

either nitrogen, sulphur or oxygen; and

$R_6$ and $R_7$ are each as defined in (a); with the proviso that when $R_3$ is a hydrogen atom, n is 0, the heterocyclic ring is an unsubstituted pyrrolidine, $R_6$ is a hydrogen atom, and $R_7$ is $OR_8$, at least one of $R_1$, $R_2$ and $R_8$ is an optionally substituted aryl radical; or

(c) $R_5$ and $R_6$ are selected to form a side chain of $\alpha$- amino acid, where $N(R_4)\ CR_5R_6COR_7$ is an $\alpha$- amino acid residue; with the proviso that (i) when $R_3$ is hydrogen and n is 0, the $\alpha$- amino acid residue is not alanine, aspartic acid, glutamic acid, glycine or phenylalanine, and (ii) when $R_3$ is hydrogen and n is 1, the $\alpha$- amino acid residue is not aspartic acid; or

(d) $R_5$ and $R_6$ are part of a cycloaklyl ring of from 3 to 6 carbon atoms; and

$R_4$ and $R_7$, where appropriate, are each as defined in (a); or a pharmaceutically acceptable salt of the compounds of the formula (I).

2. A compound according to claim 1, wherein $R_4$, $R_5$, $R_6$ and $R_7$ are each as defined in (b), with $R_6$ further being a hydrogen atom, and $R_4$ and $R_5$ further forming part of a heterocyclic ring selected from -

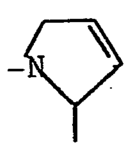 ; 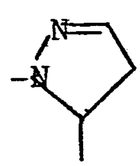 ; 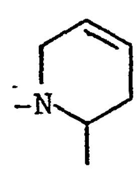 ; 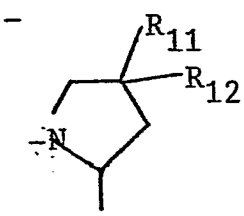

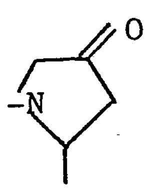 ; 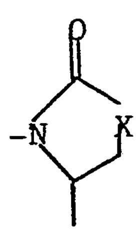 ; 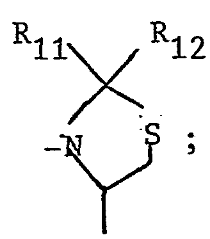 ; and 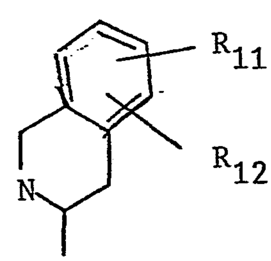

wherein X is $CH_2$, O, S, or NH and $R_{11}$ and $R_{12}$, which are the same or different, are each selected from the group consisting of a hydrogen atom, a halogen atom, a hydroxy, lower alkyl, a cycloalkyl, an optionally substituted aryl, an optionally substituted aralkyl, an alkyoxy, an aryloxy or a sulphenyl radical; the sulphur atom (when present), in the heterocyclic ring or in a substituent, being in the oxidation state of 2, 4 or 6.

3. A compound according to claim 1 or claim 2, wherein $R_4$ and $R_5$ are as defined in (b) and the heterocyclic ring is five-membered.

4. A compound according to claim 3, wherein the heterocyclic ring is a thiazolidine or pyrrolidine ring.

5. A compound according to claim 1, wherein n is 1 and the - amino acid of the residue defined in (c) is selected from arginine, phenylalanine, leucine, methionine and lysine.

6. A compound according to claim 1, wherein n is 1; at least one of $R_1$ and $R_2$ is a hydrogen atom; $R_3$ is a methyl radical; $R_4$ and $R_5$ form part of a pyrrolidine or thiazolidine ring; $R_6$ is a hydrogen atom; and/or $R_7$ forms part of a carboxylic acid or ester ($OR_8$) radical.

7. A pharmaceutically acceptable salt of a compound according to any preceding claim, being an alkali, alkaline earth metal, ammonium or quaternary ammonium salt.

8. A compound or salt according to claim 1 or claim 7, substantially as herein described with reference to any one of the specific Examples.

9. A process of preparing a compound or salt according to claim 1, wherein n is 1, comprising (I)

reacting a trialkyl or triallylphosphite of the formula

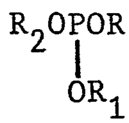

with a 2-substituted acrylic acid of the formula

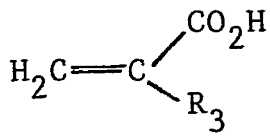

to form a triester of the formula

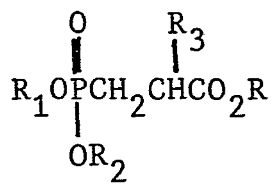 (II) ;

wherein $R_1$, $R_2$ and $R_3$ are each as defined in claim 1 and R is a lower alkyl, aryl, or aralkyl radical; (II) hydrolysing the thus formed triester with a base to form a diester of the formula

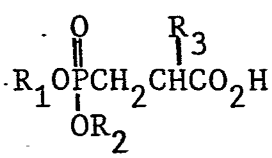 (III) ;

and (III) reacting the thus formed diester with a compound of the formula

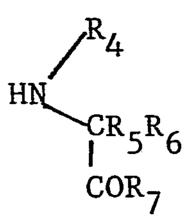 (IV) ;

in the presence of a condensing agent, thereby to form a compound according to claim 1.

10. A process according to claim 9, wherein the thus formed diester from step (II) is (IIA) converted into the acid chloride prior to reaction with the compound of formula (IV).

11.A process of preparing a compound or salt according to claim 1, wherein n is 0, comprising (I) reacting a trialkyl or triallylphosphite of the formula

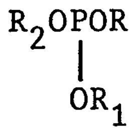

with an α - halo ester of the formula

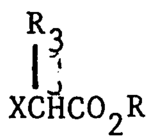

to form a triester of the formula

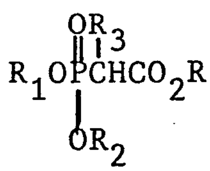
(II) ;

wherein $R_1$, $R_2$ and $R_3$ are each as defined in claim 1 and R is a lower alkyl, aryl or aralkyl radical; (II) hydrolysing the thus formed triester with a base to form a diester of the formula

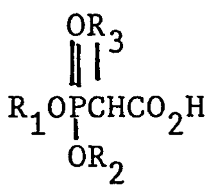
(III) ;

and (III) reacting the thus formed diester with a compound of the formula

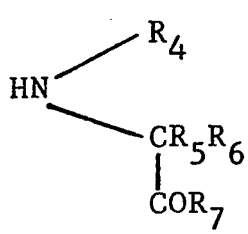
(IV) ;

in the presence of a condensing agent, thereby to form a compound according to claim 1.

12. A pharmaceutical composition comprising a compound or salt according to any one of claims 1 to 8,

together with a pharmaceutically acceptable carrier or diluent therefor.

13. A process of preparing a compound or salt according to claim 1, wherein n is 1, comprising condensing a trialkyl or triallylphosphite of the formula

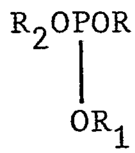

and a 2-substituted acrylic acid of the formula

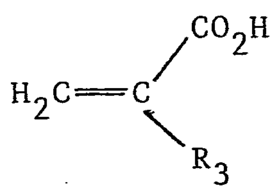

in the presence of a compound of the formula

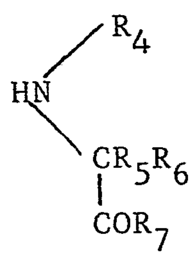

(IV)

thereby to form a compound according to claim 1.

PAUL M. TURNER
PAUL M. TURNER AND COMPANY
Chartered Patent Agent
European Patent Attorney
47 Marylebone Lane,
London W1M 6DL.

0070131

European Patent Office

# EUROPEAN SEARCH REPORT

Application number

EP 82 30 3487

## DOCUMENTS CONSIDERED TO BE RELEVANT

| Category | Citation of document with indication where appropriate, of relevant passages | Relevant to claim | CLASSIFICATION OF THE APPLICATION (Int. Cl. 3) |
|---|---|---|---|
| X | US-A-4 272 528 (A.M. VON ESCH)<br>* the whole document *<br>--- | 1-13 | C 07 F 9/38<br>C 07 F 9/65<br>A 61 K 31/675 |
| D,X | EP-A-0 000 833 (E.R. SQUIBB & SONS)<br>* the whole document *<br>--- | 1-13 | |
| D,Y | US-A-4 168 267 (E.W. PETRILLO JR.)<br>* the whole document *<br>--- | 1-13 | |
| D,Y | EP-A-0 009 183 (MERCK & CO.)<br>* the whole document *<br>----- | 1-13 | |

TECHNICAL FIELDS SEARCHED (Int. Cl. 3)

C 07 F 9/00

The present search report has been drawn up for all claims

| Place of search | Date of completion of the search | Examiner |
|---|---|---|
| THE HAGUE | 19-10-1982 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03.82